# EUROPEAN PATENT SPECIFICATION

(11) **EP 4 082 624 B1**
(45) Date of publication and mention of the grant of the patent: **23.10.2024**
(21) Application number: 20907521.7
(22) Date of filing: 28.12.2020
(51) Int. Cl.: A61Q 13/00, A61Q 15/00, A61Q 19/10, A61K 8/11, A61K 8/25, A61K 8/41, A61K 8/58, A01N 25/28, A01P 1/00, B01J 13/04, B01J 13/14, B01J 13/18, B01J 13/22, C11B 9/00, C11D 3/50, C11D 17/00

(54) **METHOD FOR PREPARING MICROCAPSULE**
VERFAHREN ZUM HERSTELLEN VON MIKROKAPSELN
PROCÉDÉ DE FABRICATION DE MICROCAPSULES

(30) Priority: 27.12.2019 JP 2019239903
(43) Date of publication of application: 02.11.2022
(73) Proprietor: Kao Corporation, Chuo-ku Tokyo 103-8210 (JP)
(72) Inventor: YAMAZAKI, Daisuke, Wakayama-shi, Wakayama 640-8580 (JP); MOROFUJI, Tatsuya, Wakayama-shi, Wakayama 640-8580 (JP); MISHIRO, Asami, Tokyo 131-8501 (JP); SAWADA, Risa, Wakayama-shi, Wakayama 640-8580 (JP); KOGA, Yoshito, Wakayama-shi, Wakayama 640-8580 (JP)
(74) Representative: Hoffmann Eitle
(86) International application number: PCT/JP2020/049258
(87) International publication number: WO 2021/132729

(56) References cited:
- EP-A1- 3 078 415
- EP-A1- 4 082 622
- EP-A1- 4 082 656
- JP-A- 2013 516 404
- JP-A- 2015 128 762
- JP-A- 2017 114 802
- US-A1- 2010 143 422
- ZHANG H ET AL: "Silica encapsulation of n-octadecane via sol-gel process: A novel microencapsulated phase-change material with enhanced thermal conductivity and performance", JOURNAL OF COLLOID AND INTERFACE SCIENCE, ACADEMIC PRESS,INC, US, vol. 343, no. 1, 1 March 2010 (2010-03-01), pages 246 - 255, XP026969417, ISSN: 0021-9797, [retrieved on 20091123]

## Description

### Field of the Invention

The present invention relates to a method for producing microcapsules.

### Background of the Invention

In a broad business field of cosmetics, medicines, general house products, prints and others, various microcapsules encapsulating a fragrance material or a physiologically active substance therein have been developed and utilized.

For example, as a shell to constitute a microcapsule, an aminoplast resin such as a melamine resin or a polyurea/urethane resin has been used. However, microcapsules are unavoidable to discharge to the environment, and in recent years, they contribute to concerns called microplastics. Consequently, it is desired to develop microcapsules having high environmental compatibility instead of aminoplast resins.

Among them, a silica microcapsule having a shell containing silica as a constituent component (hereinafter, also referred to as "silica capsule") has attracted attention as a material that can be expected to have environmental compatibility. However, in order to incorporate such silica capsules stably and for a long period of time in preparations that contain a surfactant in a high concentration such as a liquid detergent or a fabric softener, shells are required to have high denseness. In addition, preparations have different pH and viscosity depending on the use thereof, and it is also desired that silica capsules can be incorporated in preparations having a wide range of physical properties.

Silica capsules are generally synthesized by sol-gel reaction of an alkoxysilane and/or an alkylalkoxysilane as a precursor. One problem of silica capsules is that the thickness of the shell is generally thin and the denseness of the shell against a surfactant is insufficient as compared with microcapsules containing an aminoplast resin as a component of the shell. To overcome the problem, investigations for improving the denseness of shells have been made.

For example, US9532933B (PTL 1) describes a method for producing a microcapsule particle composition having an encapsulated core material inside a microcapsule shell, and describes production of a silica capsule particle composition having a strong shell by forming silica capsule particles containing a fragrance material as a core material and a cationic surfactant as an emulsifier followed by treating the silica capsule particles with polyethylene imine or the like.

JP2009-542667A (PTL 2) describes a method for preparing microcapsules having a sunscreen component or the like as a core, and describes production of capsules resistant to diffusion or transudation of an oily phase through microcapsules by using both a cationic surfactant and a nonionic surfactant and polymerizing a tetraalkoxysilane in a mode of ex-situ emulsified liquid polymerization.

JP2012-501849A (PTL 3) describes a method of improving the stability of an aqueous suspension of silicate shell microcapsules by adding a colloidal silicate sequestering agent to an aqueous suspension of silicate shell microcapsules and colloidal silicate particles.

JP2015-507522A (PTL 4) describes a method for forming active agent-containing microcapsules, and describes improvement of fragrance material retentiveness by using a fragrance material as an active agent component and a polyvinyl pyrrolidone as a polymer emulsifier and by using a blend of at least two silanes as a shell precursor.

JP2015-128762A (PTL 5) describes, for the purpose of providing a production method for microcapsules capable of retaining an organic compound of an effective ingredient such as a fragrance material for long period of time, a production method for microcapsules each having a core of an organic compound such as a fragrance material, and a first shell that encapsulates the core, and a second shell that encapsulates the first shell, which includes a first-stage sol-gel reaction where an organic phase containing an organic compound and a tetraalkoxysilane is emulsified in an aqueous phase containing a surfactant, followed by a second-stage sol-gel reaction where a tetraalkoxysilane is added to keep a pH lower than that in the first-stage sol-gel reaction, thereby producing silica capsules whose shell has high denseness.

### Summary of the Invention

The present invention relates to a method for producing microcapsules each having a shell that contains silica as a constituent component, and a core that contains at least one organic compound inside the shell, which includes:
Step 1: a step of emulsifying an aqueous phase component containing an emulsifier and an oily phase component containing at least one organic compound and a tetraalkoxysilane to give an emulsified liquid,
Step 2: a step of subjecting the emulsified liquid prepared in the step 1 to a sol-gel reaction under an acidic condition to form microcapsules (1) each having a core and a first shell containing silica as a constituent component, thereby giving an aqueous dispersion containing the microcapsules (1), and
Step 3: a step of further adding a tetraalkoxysilane to the aqueous dispersion containing the microcapsules (1) formed in the step 2, and subjecting the obtained mixture to sol-gel reaction to form microcapsules each having a second shell that encapsulates the first shell, wherein:
   the emulsifier used in the step 1 contains a cationic surfactant, and
   the content of the emulsifier in the aqueous phase component in the step 1 is 0.30% by mass or less.

### Detailed Description of the Invention

According to the techniques of PTLs 1 to 5, when silica capsules are incorporated in a preparation containing a surfactant in a high concentration, the encapsulated component such as a fragrance material cannot be retained for a long period of time, and the dispersion stability of the silica capsules in various preparations is insufficient.

The technique of PTL 1 is for improving the denseness of shells by posttreatment after formation of silica capsule particles, while in the technique of PTL 3 where a colloidal silicate sequestering agent is not added, gelation occurs during high-temperature storage to give an unstable suspension, and according to these techniques, application of silica capsules to preparations is limited. The technique of PTL 2 where an organic compound such as a fragrance material that has a smaller oil/water surface tension and is more unstable in emulsification than a sunscreen component is used as an encapsulated component is still insufficient in point of long-term retentiveness of the encapsulated component.

The present invention relates to a method for producing microcapsules capable of retaining the encapsulated organic compound such as a fragrance material for a long period of time and excellent in dispersion stability even in various preparations.

The present inventors have found that, when the emulsifier for use for an emulsified liquid for sol-gel reaction contains a cationic surfactant and when the content of the emulsifier in the aqueous phase component of the emulsified liquid is controlled to be a predetermined amount or less, then silica capsules whose shell can have high denseness while securing stability of emulsified drops can be obtained. Further, the present inventors have noted that the resultant silica capsules are stably dispersible without gelation and can be stably blended in any preparations of acidic preparations, neutral preparations and alkaline preparations, and have found that there can be provided a method for producing microcapsules capable of retaining an encapsulated organic compound such as a fragrance material for a long period of time and excellent in dispersion stability in various preparations.

Specifically, the present invention relates to a method for producing microcapsules each having a shell that contains silica as a constituent component, and a core that contains at least one organic compound inside the shell, which includes:
Step 1: a step of emulsifying an aqueous phase component containing an emulsifier and an oily phase component containing at least one organic compound and a tetraalkoxysilane to give an emulsified liquid,
Step 2: a step of subjecting the emulsified liquid prepared in the step 1 to a sol-gel reaction under an acidic condition to form microcapsules (1) each having a core and a first shell containing silica as a constituent component, thereby giving an aqueous dispersion containing the microcapsules (1), and
Step 3: a step of further adding a tetraalkoxysilane to the aqueous dispersion containing the microcapsules (1) formed in the step 2, and subjecting the obtained mixture to sol-gel reaction to form microcapsules each having a second shell that encapsulates the first shell, wherein:
   the emulsifier used in the step 1 contains a cationic surfactant, and
   the content of the emulsifier in the aqueous phase component in the step 1 is 0.30% by mass or less.

According to the present invention, there can be provided a method for producing microcapsules capable of retaining an encapsulated organic compound such as a fragrance material for a long period of time and excellent in dispersion stability in various preparations.

### [Production Method for Microcapsules]

The production method for microcapsules of the present invention is a method for producing microcapsules (silica capsules) each having a shell that contains silica as a constituent component, and a core that contains at least one organic compound inside the shell, which includes:
Step 1: a step of emulsifying an aqueous phase component containing an emulsifier and an oily phase component containing at least one organic compound and a tetraalkoxysilane to give an emulsified liquid,
Step 2: a step of subjecting the emulsified liquid prepared in the step 1 to a sol-gel reaction under an acidic condition to form microcapsules (1) (silica capsules (1)) each having a core and a first shell containing silica as a constituent component, thereby giving an aqueous dispersion containing the silica capsules (1), and
Step 3: a step of further adding a tetraalkoxysilane to the aqueous dispersion containing the silica capsules (1) formed in the step 2, and subjecting the obtained mixture to sol-gel reaction to form silica capsules each having a second shell that encapsulates the first shell, wherein:
   the emulsifier used in the step 1 contains a cationic surfactant, and the content of the emulsifier in the aqueous phase component in the step 1 is 0.30% by mass or less.

In the present invention, the "sol-gel reaction" means a reaction of forming silica that is a constituent component of a shell by hydrolysis and polycondensation reaction of a tetraalkoxysilane (namely, silica precursor) via a sol and gel state. Specifically, a tetraalkoxysilane is hydrolyzed and the silanol compound forms a siloxane oligomer by dehydrating condensation reaction and dealcoholating condensation reaction of silanol compound, and via further dehydrating condensation reaction, silica is formed.

In the present invention, "encapsulates the first shell" means to encapsulate the first shell of the silica capsules (1) formed in the step 2, and also includes encapsulating the silica capsules (1).

Also in the present invention, the first shell formed in the step 2 is referred to as "the first shell", and the second shell formed in the step 3 is referred to as "the second shell".

In the present specification, the long-term retentiveness of the encapsulated organic compound may be referred to as "long-term retentiveness".

According to the production method of the present invention, there can be produced silica capsules capable of retaining an organic compound of an effective component such as a fragrance material for a long period of time and excellent in dispersion stability in various preparations. Though not clear, the reason can be considered as follows.

The kind and the amount of the emulsifier in the emulsified liquid to be subjected to sol-gel reaction are considered to have a great influence on the dispersion stability of the emulsified liquid containing the encapsulated organic compound and tetraalkoxysilane and on the denseness of the silica shell formed in the subsequent step 2 and step 3. With that, it is considered that the cationic surfactant promotes silica shell formation, but by performing the sol-gel reaction using a smaller amount of the cationic surfactant, the denseness of the silica shells to constitute the resultant silica capsules can improve. Consequently, when the emulsifier used in the step 1 contains a cationic surfactant and when the content of the emulsifier in the aqueous phase component is controlled to be a predetermined value or less, then the number of the emulsifier micelles not containing an organic compound in the aqueous phase can be reduced, the adsorption amount of silica sol to the emulsifier micelles can be reduced, and the silica sol can be efficiently adsorbed to the emulsion drops, and accordingly as a result, the shell denseness can be thereby improved.

In addition, when the content of the emulsifier in the aqueous phase component is controlled to be a predetermined value or less, formation of emulsifier micelles can be prevented and formation of colloidal silica caused by adsorption of silica sol to the emulsifier micelles can be thereby suppressed. As a result, it is also considered that gelation caused by instability of colloidal silica, for example, owing to alkali addition can also be suppressed and good dispersion stability in various preparations can be thereby maintained.

### (Step 1)

The step 1 is a step of emulsifying an aqueous phase component containing an emulsifier and an oily phase component containing at least one organic compound and a tetraalkoxysilane to give an emulsified liquid.

### <Emulsifier>

The emulsifier used in the step 1 contains a cationic surfactant.

The cationic surfactant includes an alkylamine salt, and an alkyl quaternary ammonium salt. The carbon number of alkyl group of the alkylamine salt and the alkyl quaternary ammonium salt is preferably 10 or more, more preferably 12 or more, even more preferably 14 or more, and is preferably 22 or less, more preferably 20 or less, even more preferably 18 or less.

The alkylamine salt includes an alkylamine acetate such as laurylamine acetate and stearylamine acetate.

The quaternary ammonium salt incudes an alkyltrimethylammonium salt, a dialkyldialkylammonium salt and an alkylbenzyldimethylammonium salt.

The alkyltrimethylammonium salt includes an alkyltrimethylammonium chloride such as lauryltrimethylammonium chloride, cetyltrimethylammonium chloride and stearyltrimethylammonium chloride; and an alkyltrimethylammonium bromide such as lauryltrimethylammonium bromide, cetyltrimethylammonium bromide and stearyltrimethylammonium bromide.

The dialkyldimethylammonium salt includes a dialkyldimethylammonium chloride such as distearyldimethylammonium chloride; and a dialkyldimethylammonium bromide such as distearyldimethylammonium bromide.

The alkylbenzyldimethylammonium salt includes an alkylbenzyldimethylammonium chloride and an alkylbenzyldimethylammonium bromide.

Among these, the cationic surfactant is preferably a quaternary ammoniums salt, more preferably an alkyltrimethylammonium salt, even more preferably an alkyltrimethylammonium chloride, further more preferably at least one selected from the group consisting of lauryltrimethylammonium chloride, stearyltrimethylammonium chloride and cetyltrimethylammonium chloride, further more preferably cetyltrimethylammonium chloride.

The emulsifier for use in the step 1 may further contain any other emulsifier in addition to the cationic surfactant, within a range not detracting from the advantageous effects of the present invention. The other emulsifier includes a polymer dispersant, a nonionic surfactant, an anionic surfactant, and an ampholytic surfactant. Above all, at least one selected from the group consisting of a polymer dispersant and a nonionic surfactant is preferred.

The polymer dispersant includes, from the viewpoint of adsorbing to an oil/water interface and a solid/liquid interface to improve the dispersion stability of emulsified drops, a polyvinyl alcohol; a polyvinyl pyrrolidone; and a cellulosic polymer, such as methyl cellulose and ethyl cellulose, a hydroxyalkyl cellulose such as hydroxyethyl cellulose, hydroxypropyl cellulose and hydroxypropylmethyl cellulose, and a carboxyalkyl cellulose such as carboxymethyl cellulose and carboxyethyl cellulose.

The nonionic surfactant includes a polyoxyethylene alkyl ether having 8 or more and 22 or less carbon atoms; a polyoxyethylene alkyl phenyl ether having 8 or more and 22 or less carbon atoms; a polyoxyethylene polyoxypropylene glycol having 8 or more and 22 or less carbon atoms; a sorbitan fatty acid ester such as sorbitan monostearate and sorbitan monopalmitate; and a sucrose fatty acid ester such as a sugar ester.

In the step 1, the content of the cationic surfactant in the emulsifier is, from the viewpoint of promoting shell formation in the step 2 to improve long-term retentiveness and dispersion stability of silica capsules, preferably 70% by mass or more, more preferably 80% by mass or more, even more preferably 90% by mass or more, further more preferably 95% by mass or more, and is preferably 100% by mass or less, more preferably 100% by mass.

In the step 1, the content of the polymer dispersant in the emulsifier is, from the viewpoint of promoting shell formation in the step 2 to improve long-term retentiveness and dispersion stability of silica capsules, preferably 30% by mass or less, more preferably 20% by mass or less, even more preferably 10% by mass or less, further more preferably 5% by mass or less, and is further more preferably 0% by mass, that is, preferably, a polymer dispersant is not added in the step 1.

In the case of using a polymer dispersant, from the viewpoint of promoting shell formation in the step 2 to improve long-term retentiveness and dispersion stability of silica capsules, preferably the polymer dispersant is added in and after the step 3 to be mentioned hereinunder.

In the step 1, the content of the emulsifier in the aqueous phase component is, from the viewpoint of suppressing formation of emulsifier micelles not containing an organic compound owing to the excessive emulsifier not contributing to the dispersion stability of an emulsified liquid, to thereby promote shell formation, 0.30% by mass or less, preferably 0.28% by mass or less, more preferably 0.25% by mass or less, even more preferably 0.23% by mass or less, further more preferably 0.20% by mass or less, and is, from the viewpoint of dispersion stability of emulsified drops, preferably 0.05% by mass or more, more preferably 0.07% by mass or more, even more preferably 0.10% by mass or more, further more preferably 0.15% by mass or more, further more preferably 0.17% by mass or more.

In the step 1, the content of the cationic surfactant in the aqueous phase component is, from the viewpoint of dispersion stability of emulsified drops, preferably 0.05% by mass or more, more preferably 0.07% by mass or more, even more preferably 0.10% by mass or more, further more preferably 0.15% by mass or more, further more preferably 0.17% by mass or more, and is, from the viewpoint of suppressing formation of emulsifier micelles owing to the excessive emulsifier not contributing to the dispersion stability of an emulsified liquid, to thereby improve encapsulation efficiency, preferably 0.30% by mass or less, more preferably 0.28% by mass or less, even more preferably 0.25% by mass or less, further more preferably 0.23% by mass or less, further more preferably 0.20% by mass or less.

The amount to be added of the emulsifier is, from the viewpoint of forming a stable emulsified liquid, preferably 0.1 parts by mass or more relative to 100 parts by mass of the oily phase component used in the step 1, more preferably 0.15 parts by mass or more, even more preferably 0.20 parts by mass or more, and is preferably 5 parts by mass or less, more preferably 4 parts by mass or less, even more preferably 3 parts by mass or less, further more preferably 2 parts by mass or less, further more preferably 1 part by mass or less.

### <Organic Compounds

The core of the silica capsule in the present invention contains at least one organic compound. The organic compound is preferably at least one material selected from the group consisting of a fragrance material, a fragrance precursor, an oil (e.g., a moisturizer), an antioxidant, an antibacterial agent, a fertilizer, fibers (e.g., cloth), a surface modifier for skin and hair, a cooling sensation agent, a dye, a colorant, a silicone, a solvent, and an oil-soluble polymer, more preferably at least one material selected from the group consisting of a fragrance material, a fragrance precursor, an oil, an antioxidant, an antibacterial agent, a fertilizer, a surface modifier, and a solvent, even more preferably at least one material selected from the group consisting of a fragrance material, a fragrance modifier, an oil, an antioxidant and a solvent, further more preferably at least one material selected from the group consisting of a fragrance material, a fragrance precursor and an oil, further more preferably at least one material selected from the group consisting of a fragrance material, a fragrance precursor and a moisturizer, further more preferably at least one material selected from the group consisting of a fragrance material and a fragrance precursor.

One alone or two or more kinds of the organic compounds can be used either singly or as combined.

The fragrance precursor includes a compound that reacts with water to release a fragrance component, and a compound that reacts with light to release a fragrance component.

The compound that reacts with water to release a fragrance component includes a silicate ester compound having an alkoxy component derived from a fragrance alcohol, a fatty acid ester compound having an alkoxy component derived from a fragrance alcohol, an acetal compound or a hemiacetal compound obtained by reaction of a carbonyl component derived from a fragrance aldehyde or a fragrance ketone and an alcohol compound, a Schiff base compound obtained by reaction of a carbonyl component derived from a fragrance aldehyde or a fragrance ketone and a primary amine compound, and a hemiaminal compound or hydrazone compound obtained by reaction of a carbonyl component derived from a fragrance aldehyde or a fragrance ketone and a hydrazine compound.

The compound that reacts with light to release a fragrance component includes a 2-nitrobenzyl ether compound having an alkoxy component derived from a fragrance alcohol, an α-keto-ester compound having a carbonyl component derived from a fragrance aldehyde or fragrance ketone, and a coumaric acid ester compound having an alkoxy compound derived from a fragrance alcohol. These fragrance precursors can be used as a polymer of, for example, a reaction product of some carboxy groups of a polyacrylic acid and a fragrance alcohol.

From the viewpoint of forming a stable emulsified liquid, the organic compound preferably has appropriate hydrophobicity. As an index to indicate the hydrophilicity or the hydrophobicity of the organic compound, employable is a cLogP value that is a calculated value of a common logarithm "logP" of a partition coefficient P between n-octanol and water (n-octanol/water). The cLogP value is a "LogP (cLogP)" that is calculated according to the method described in A. Leo Comprehensive Medicinal Chemistry, Vol. 4, C. Hansch, P. G. Sammens, J. B Taylor and C. A. Ramsden, Eds., p. 295, Pergamon Press, 1990, and is a cLogP value calculated by Program CLOGP v. 4.01.

In the case where the organic compound is composed of plural constituent components, the cLogP value of the organic compound can be determined by multiplying the cLogP value of each constituent component by the volume ratio thereof, and summing up the resultant data.

The cLogP value of the organic compound is preferably 1 or more, more preferably 2 or more, even more preferably 3 or more, further more preferably 4 or more, and is preferably 30 or less, more preferably 20 or less, even more preferably 10 or less.

When the cLogP value of the organic compound is 1 or more, the encapsulation ratio of the organic compound in the silica capsules to be formed in the sol-gel reaction of the oil-in-water drops to be mentioned below (hereinafter this may also be referred to as "encapsulation ratio") increases. Also in the case where the organic compound is a fragrance material composition composed of plural fragrance material components and when the cLogP value of the fragrance material composition is 1 or more, the encapsulation ratio of the fragrance material composition in the silica capsules to be formed in the sol-gel reaction can also increase.

The amount of the oily phase component relative to the total amount of the emulsified liquid formed in the step 1 is, from the viewpoint of production efficiency, preferably 5% by mass or more, more preferably 10% by mass or more, even more preferably 15% by mass or more, further more preferably 20% by mass or more, and is, from the viewpoint of forming a stable emulsified liquid, preferably 70% by mass or less, more preferably 60% by mass or less, even more preferably 50% by mass or less.

### <Tetraalkoxysilane>

The tetraalkoxysilane for use in the step 1 is, from the viewpoint of promoting sol-gel reaction, preferably has an alkoxy group having 1 or more and 4 or less carbon atoms, and is more preferably at least one selected from the group consisting of tetramethoxysilane, tetraethoxysilane and tetraisopropoxysilane, even more preferably at least one selected from the group consisting of tetramethoxysilane and tetraethoxysilane, further more preferably tetraethoxysilane.

The amount to be added of the tetraalkoxysilane in the step 1 is, from the viewpoint of forming a shell enough to sufficiently encapsulate the surfaces of the emulsified drops containing an organic compound, preferably 10% by mass or more relative to the amount of the organic compound in the step 1, more preferably 15% by mass or more, even more preferably 20% by mass or more, and is, from the viewpoint of suppressing residues inside the oil drops to reduce the amount of excessive tetraalkoxysilane not contributing to shell formation, preferably 100% by mass or less, more preferably 70% by mass or less, even more preferably 50% by mass or less, further more preferably 40% by mass or less, further more preferably 30% by mass or less.

The step 1 preferably includes the following step 1-1 to step 1-3.

Step 1-1: a step of preparing an aqueous phase component containing an emulsifier.

Step 1-2: a step of mixing at least one organic compound and a tetraalkoxysilane to prepare an oily phase component.

Step 1-3: a step of mixing and emulsifying the aqueous phase component prepared in the step 1-1 and the oily phase component prepared in the step 1-2 to give an emulsified liquid.

Not specifically limited, the stirring means for use in mixing and emulsifying the aqueous phase component and the oily phase component may be a homogenizer having a strong shear force, a high-pressure disperser, and an ultrasonic disperser. In addition, a homomixer, "DISPER" (tradename, by PRIMIX Corporation), "CLEAMIX" (tradename, by M Technique Co., Ltd.), and "CAVITRON" (tradename, by Pacific Machinery & Engineering Co., Ltd.) can also be used.

The median diameter D₅₀ of the emulsified drops in the emulsified liquid formed in the step 1 is, from the viewpoint of reducing the specific surface area relative to the environment outside the silica capsules to improve long-term retentiveness, preferably 0.1 pm or more, more preferably 0.2 pm or more, even more preferably 0.3 pm or more, further more preferably 0.5 pm o more, further more preferably 0.8 pm or more, and is, from the viewpoint of the physical strength of silica capsules, preferably 50 pm or less, more preferably 30 pm or less, even more preferably 10 pm or less, further more preferably 5 pm or less, further more preferably 3 pm or less, further preferably less than 2 pm.

The median diameter D₅₀ of the emulsified drops can be measured according to the method described in the section of Examples.

### (Step 2)

The step 2 is a step of subjecting the emulsified liquid prepared in the step 1 to a sol-gel reaction under an acidic condition to form silica capsules (1) each having a core and a first shell containing silica as a constituent component, thereby giving an aqueous dispersion containing the silica capsules (1).

The initial pH in the sol-gel reaction in the step 2 is, from the viewpoint of keeping the balance between hydrolysis reaction and condensation reaction of tetraalkoxysilane and from the viewpoint of suppressing formation of a sol having high hydrophilicity to promote encapsulation, preferably 3.0 or more, more preferably 3.3 or more, even more preferably 3.5 or more, and is, from the viewpoint of suppressing parallel occurrence of silica shell formation and aggregation of emulsified drops to give silica capsules each having a dense shell, preferably 4.5 or less, more preferably 4.3 or less, even more preferably 4.1 or less.

From the viewpoint of controlling the desired initial pH depending on the acidic or alkaline intensity of the oily phase component containing an organic compound, an arbitrary acidic or alkaline pH regulator can be used.

pH of the emulsified liquid could not reach a desired value. In such a case, preferably, an alkaline pH regulator to be mentioned below is used for pH control.

Specifically, the step 2 may preferably include the following step 2-1.

Step 2-1: a step of controlling the pH of the emulsified liquid prepared in the step 1, using a pH regulator.

The acidic pH regulator includes an inorganic acid such as hydrochloric acid, nitric acid, and sulfuric acid, an organic acid such as acetic acid, and citric acid, and a liquid prepared by adding a cation exchange resin in water or methanol, and is preferably hydrochloric acid, sulfuric acid, nitric acid, or citric acid.

The alkaline pH regulator includes sodium hydroxide, sodium hydrogencarbonate, potassium hydroxide, ammonium hydroxide, diethanolamine, triethanolamine, and trishydroxymethylaminomethane, and is preferably sodium hydroxide or ammonium hydroxide.

The reaction temperature of the sol-gel reaction in the step 2 can be arbitrarily selected in a range of the melting point of water contained as the aqueous phase or higher and the boiling point thereof or lower, but is, from the viewpoint of controlling the balance between hydrolysis reaction and condensation reaction in the sol-gel reaction to form a dense shell, preferably 5°C or higher, more preferably 10°C or higher, even more preferably 15°C or higher, further more preferably 20°C or higher, and is preferably 60°C or lower, more preferably 50°C or lower, even more preferably 40°C or lower.

### (Step 3)

The step 3 is a step of further adding a tetraalkoxysilane to the aqueous dispersion containing the silica capsules (1) formed in the step 2, and subjecting the obtained mixture to sol-gel reaction to form silica capsules each having a second shell that encapsulates the first shell.

### <Tetraalkoxysilane>

Tetraalkoxysilane for use in the step 3 is, from the viewpoint of promoting sol-gel reaction, preferably one having an alkoxy group having 1 or more and 4 or less carbon atoms, more preferably at least one selected from the group consisting of tetramethoxysilane, tetraethoxysilane and tetraisopropoxysilane, even more preferably at least one selected from the group consisting of tetramethoxysilane and tetraethoxysilane, further more preferably tetraethoxysilane.

The amount to be added of the tetraalkoxysilane in the step 3 is, from the viewpoint of forming the second shell to encapsulate the first shell, preferably 5% by mass or more relative to the amount of the organic compound in the step 1, more preferably 7% by mass or more, even more preferably 10% by mass or more, and is, from the viewpoint of suppressing formation of silica sol to disperse in the aqueous phase to improve the dispersion stability of silica capsule, preferably 100% by mass or less, mor preferably 70% by mass or less, even more preferably 50% by mass or less, further more preferably 30% by mass or less, further more preferably 20% by mass or less.

In the step 3, the tetraalkoxysilane to be added to the aqueous dispersion containing the silica capsules (1) formed in the step 2 can be added all at a time, or can be added intermittently as divided in portions, or can be added continuously, but from the viewpoint of forming the second shell having high denseness, it is preferred that it is continuously dropwise added.

In the case where tetraalkoxysilane is continuously dropwise added, the dropwise addition time can be appropriately set depending on the production scale, but is, from the viewpoint of preventing separation of the tetraalkoxysilane added and the aqueous dispersion, preferably 5 minutes or more, more preferably 10 minutes or more, even more preferably 30 minutes or more, and is, from the viewpoint of shortening the reaction time to increase the denseness of the second shell to be formed, preferably 1200 minutes or less, more preferably 1000 minutes or less, even more preferably 500 minutes or less.

The reaction temperature of the sol-gel reaction in the step 3 can be arbitrarily selected in a range of the melting point of water contained as the dispersion medium or higher and the boiling point thereof or lower, but is, from the viewpoint of controlling the balance between hydrolysis reaction and condensation reaction in the sol-gel reaction to form a dense shell, preferably 5°C or higher, more preferably 10°C or higher, even more preferably 15°C or higher, further more preferably 20°C or higher, and is preferably 60°C or lower, more preferably 50°C or lower, even more preferably 40°C or lower.

The sol-gel reaction in the step 2 and the sol-gel reaction in the step 3 can be carried out at the same reaction temperature or can be carried out at different reaction temperatures.

### [Microcapsules]

The microcapsules (silica capsules) in the present invention are obtained as an aqueous dispersion thereof dispersed in water. In accordance with the use of the silica capsules, the aqueous dispersion can be used directly as it is, but depending on the use thereof, the silica capsules can be separated from the aqueous dispersion. As the method for separation, a filtration method or a centrifugal method can be employed.

The silica capsules in the present invention are silica capsules each having a core that contains the above-mentioned organic compound, a first shell that encapsulates the core, and a second shell that encapsulates the first shell.

The first shell of the silica capsules in the present invention encapsulates a core and contains silica as a constituent component and preferably has an average thickness of 5 nm or more and 20 nm or less, and the second shell encapsulates the first shell and contains silica as a constituent component and preferably has an average thickness of 10 nm or more and 100 nm or less.

The average thickness of the first shell and the second shell of the silica capsules in the present invention can be measured by transmission electron microscopy (TEM). Specifically, under observation with a transmission electron microscope, the thickness of the first shell and the second shell is measured on the photographic image. This operation is carried out in different five view fields. From the resultant data, the average thickness distribution of the first shell and the second shell is determined. As a rough indication, the transmission electron microscope has a magnification of 10,000 times or more and 100,000 times or less, but can be appropriately controlled depending on the size of the silica capsules. Here, as a transmission electron microscope (TEM), for example, "JEM-2100" (tradename by JEOL Corporation) can be used.

The median diameter D₅₀ of the silica capsules in the present invention is, from the viewpoint of improving long-term retentiveness and improving the dispersion stability of the silica capsules, preferably 0.1 pm or more, more preferably 0.5 pm or more, even more preferably 1 pm or more, and is, from the viewpoint of improving the physical strength of the silica capsules to improve long-term retentiveness, preferably 50 pm or less, more preferably 10 pm or less, even more preferably 3 pm or less.

The median diameter D₅₀ of the silica capsules can be measured according to the method described in the section of Examples.

The silica capsules in the present invention can be suitably used in various applications, for example, as cosmetic materials such as milk, cosmetic fluid, lotion, essence liquid, cream, gel formulation, hair treatment agent, and quasi-drugs, fiber treatment agents such as detergent, softener, and antiwrinkle spray, hygiene products such as paper diaper, and aromatic materials.

The silica capsules in the present invention can be used as contained or blended in a composition such as a detergent composition, a fiber treatment composition, a cosmetic composition, an aromatic composition, and a deodorant composition. The composition is preferably a detergent composition such as a powdery detergent composition, and a liquid detergent composition; and a fiber treatment composition such as a softener composition, more preferably a fiber treatment composition, even more preferably a softener composition.

Regarding the above-mentioned embodiments, the present invention further discloses the following production method for microcapsules.
<1> A method for producing microcapsules each having a shell that contains silica as a constituent component, and a core that contains at least one organic compound inside the shell, which includes:
   Step 1: a step of emulsifying an aqueous phase component containing an emulsifier and an oily phase component containing at least one organic compound and a tetraalkoxysilane to give an emulsified liquid,
   Step 2: a step of subjecting the emulsified liquid prepared in the step 1 to a sol-gel reaction under an acidic condition to form microcapsules (1) each having a core and a first shell containing silica as a constituent component, thereby giving an aqueous dispersion containing the microcapsules (1), and
   Step 3: a step of further adding a tetraalkoxysilane to the aqueous dispersion containing the microcapsules (1) formed in the step 2, and subjecting the obtained mixture to sol-gel reaction to form microcapsules each having a second shell that encapsulates the first shell, wherein:
      the emulsifier used in the step 1 contains a cationic surfactant, and
      the content of the emulsifier in the aqueous phase component in the step 1 is 0.30% by mass or less.
<2> The method for producing microcapsules according to <1>, wherein the cationic surfactant is preferably a quaternary ammonium salt.
<3> The method for producing microcapsules according to <1> or <2>, wherein the cationic surfactant is preferably an alkyltrimethylammonium salt.
<4> The method for producing microcapsules according to any of <1> to <3>, wherein the cationic surfactant is preferably an alkyltrimethylammonium chloride.
<5> The method for producing microcapsules according to any of <1> to <4>, wherein the cationic surfactant is preferably at least one selected from the group consisting of lauryltrimethylammonium chloride, stearyltrimethylammonium chloride and cetyltrimethylammonium chloride.
<6> The method for producing microcapsules according to any of <1> to <5>, wherein the cationic surfactant is preferably cetyltrimethylammonium chloride.
<7> The method for producing microcapsules according to any of <1> to <6>, wherein the content of the cationic surfactant in the aqueous phase component in the step 1 is 0.07% by mass or more and 0.28% by mass or less.
<8> The method for producing microcapsules according to any of <1> to <7>, wherein the content of the cationic surfactant in the aqueous phase component in the step 1 is 0.10% by mass or more and 0.25% by mass or less.
<9> The method for producing microcapsules according to any of <1> to <8>, wherein the content of the cationic surfactant in the aqueous phase component in the step 1 is 0.15% by mass or more and 0.23% by mass or less.
<10> The method for producing microcapsules according to any of <1> to <9>, wherein the content of the cationic surfactant in the aqueous phase component in the step 1 is 0.17% by mass or more and 0.20% by mass or less.
<11> The method for producing microcapsules according to any of <1> to <10>, wherein the amount to be added of the emulsifying agent is preferably 0.1 parts by mass or more and 5 parts by mass or less relative to 100 parts by mass of the oily phase component used in the step 1.
<12> The method for producing microcapsules according to any of <1> to <11>, wherein the amount to be added of the emulsifying agent is preferably 0.15 parts by mass or more and 4 parts by mass or less relative to 100 parts by mass of the oily phase component used in the step 1.
<13> The method for producing microcapsules according to any of <1> to <12>, wherein the amount to be added of the emulsifying agent is preferably 0.20 parts by mass or more and 3 parts by mass or less relative to 100 parts by mass of the oily phase component used in the step 1.
<14> The method for producing microcapsules according to any of <1> to <13>, wherein the cLogP value of the organic compound is preferably 1 or more and 30 or less.
<15> The method for producing microcapsules according to any of <1> to <14>, wherein the cLogP value of the organic compound is preferably 2 or more and 20 or less.
<16> The method for producing microcapsules according to any of <1> to <15>, wherein the cLogP value of the organic compound is preferably 3 or more and 10 or less.
<17> The method for producing microcapsules according to any of <1> to <16>, wherein the initial pH in the sol-gel reaction in the step 2 is preferably 3.3 or more and 4.3 or less.
<18> The method for producing microcapsules according to any of <1> to <17>, wherein the initial pH in the sol-gel reaction in the step 2 is preferably 3.5 or more and 4.1 or less.
<19> The method for producing microcapsules according to any of <1> to <18>, wherein the amount to be added of tetraalkoxysilane in the step 3 is preferably 5% by mass or more and 30% by mass or less relative to the amount of the organic compound in the step 1.
<20> The method for producing microcapsules according to any of <1> to <19>, wherein the amount to be added of tetraalkoxysilane in the step 3 is preferably 5% by mass or more and 20% by mass or less relative to the amount of the organic compound in the step 1.
<21> The method for producing microcapsules according to any of <1> to <20>, wherein the amount to be added of tetraalkoxysilane in the step 3 is preferably 7% by mass or more and 20% by mass or less relative to the amount of the organic compound in the step 1.
<22> The method for producing microcapsules according to any of <1> to <21>, wherein the median diameter D₅₀ of the silica capsules is preferably 0.1 pm or more and 50 pm or less.
<23> The method for producing microcapsules according to any of <1> to <22>, wherein the median diameter D₅₀ of the silica capsules is preferably 0.1 pm or more and 10 pm or less.
<24> The method for producing microcapsules according to any of <1> to <23>, wherein the median diameter D₅₀ of the silica capsules is preferably 0.1 pm or more and 3 pm or less.

### Examples

Various measurements in Examples and Comparative Examples were according to the following methods.

### [Median Diameter D₅₀]

The median diameter D₅₀ of emulsified drops and the median diameter D₅₀ of silica capsules were measured using a laser diffraction/scattering particle size distribution analyzer "LA-950" (trade name by Horiba, Ltd.). The measurement was performed using a flow cell, and water was used as a medium. A refractive index was set to 1.45-0-i for a dispersoid. An emulsion or an aqueous dispersion containing silica capsules was added to the flow cell, and the measurement was carried out at a concentration at which a transmittance thereof was near 90%, to determine the median diameter D₅₀ based on the volume.

### <Model Fragrance Material>

A model fragrance material A (volume-average cLogP: 4.2. specific gravity: 0.96), a model fragrance material B (volume-average cLogP: 3.5, specific gravity: 0.87) and a model fragrance material C (volume-average cLogP: 4.4, specific gravity: 0.94) each having the composition shown in Table 1 to Table 3, respectively, were used as the organic compound to be encapsulated in silica capsules. The volume-average cLogP value of the model fragrance material was calculated by multiplying the cLogP values of all the fragrance components in the model fragrance material by the volume ratio thereof in the model fragrance material and summing up the resultant data

**Table 1: Model Fragrance Material A**

| Fragrance Component Name | Content (mass%) | cLogP |
|---|---|---|
| Hexyl Acetate | 10 | 2.8 |
| Tetrahydrolinalol | 10 | 3.6 |
| Hexylcinnamaldehyde | 20 | 4.9 |
| Methyl Dihydrojasmonate | 10 | 3.0 |
| α-ionone | 10 | 3.9 |
| Lilial | 20 | 4.4 |
| Hexyl Salicylate | 20 | 5.1 |

**Table 2: Model Fragrance Material B**

| Fragrance Component Name | Content (mass%) | cLogP |
|---|---|---|
| Linalol | 22 | 3.3 |
| Linalyl Acetate | 16 | 4.4 |
| Tetrahydrolinalol | 16 | 3.6 |
| Caryophyllene | 5 | 6.3 |
| Coumarin | 4 | 1.5 |
| Eucalyptus Oil | 3 | 3.1 |
| Isobornyl Acetate | 3 | 3.9 |
| Ocimene | 3 | 4.8 |
| Borneol | 3 | 2.9 |
| Nellyl Acetate | 2 | 4.5 |
| Alphapinene | 2 | 4.3 |
| Cis-3-hexenol | 2 | 1.6 |
| Others | 19 | |

**Table 3: Model Fragrance Material C**

| Fragrance Component Name | Content (mass%) | cLogP |
|---|---|---|
| Limonene | 25 | 3.5 |
| Methyl Eugenol | 25 | 3.0 |
| Hexylcinnamylaldehyde | 50 | 4.9 |

### Example 1

### (Step 1)

0.60 g of ΓaUARTAMIN 60W (tradename by Kao Corporation, cetyltrimethylammonium chloride (hereinafter expressed as "CTAC"), active ingredient: 30% by mass) was diluted with 89.14 g of ion-exchanged water to prepare an aqueous phase component. An oily phase component prepared by mixing 24 g of the model fragrance material A and 6 g of tetraethoxysilane (hereinafter expressed as "TEOS") was added to the aqueous phase component, and the mixture liquid was emulsified using a homomixer (by HsiangTai, Model: HM-310) set at a rotation number of 8,500 rpm to give an emulsified liquid. At that time, the median diameter D₅₀ of the emulsified drops was 1.1 pm.

### (Step 2)

The emulsified liquid prepared in the step 1 was controlled to have a pH of 3.8 using 0.26 g of an aqueous solution of 0.1 N sodium hydroxide, then transferred to a separable flask equipped with a stirring blade and a cooling device, and while the liquid temperature was kept at 30°C, this was stirred at 200 rpm for 24 hours to give an aqueous dispersion containing silica capsules (1-1) each having a core of the model fragrance material A and a first shell of silica.

### (Step 3)

90.0 g was sampled from the total, 120 g of the aqueous dispersion prepared in the step 2, and while this was kept stirred at a liquid temperature of 30°C, 2.7 g of TEOS was dropwise added thereto taking 420 minutes. After the dropwise addition, this was kept stirred for further 17 hours, and then cooled to form a second shell encapsulating the first shell, thereby giving an aqueous dispersion containing silica capsules (A-1) where the model fragrance material A was encapsulated with amorphous silica. The median diameter D₅₀ of the silica capsules (A-1) was 2.1 pm.

### Example 2

### (Step 1)

0.90 g of ΓaUARTAMIN 60W was diluted with 89.10 g of ion-exchanged water to prepare an aqueous phase component. An oily phase component prepared by mixing 24 g of the model fragrance material A and 6 g of TEOS was added to the aqueous phase component, and the mixture liquid was emulsified using the above-mentioned homomixer set at a rotation number of 8,500 rpm to give an emulsified liquid. At that time, the median diameter D₅₀ of the emulsified drops was 1.1 µm.

### (Step 2)

The emulsified liquid prepared in the step 1 was controlled to have a pH of 3.8 using 0.26 g of an aqueous solution of 0.1 N sodium hydroxide, then transferred to a separable flask equipped with a stirring blade and a cooling device, and while the liquid temperature was kept at 30°C, this was stirred at 200 rpm for 24 hours to give an aqueous dispersion containing silica capsules (1-2) each having a core of the model fragrance material A and a first shell of silica.

### (Step 3)

90.0 g was sampled from the total, 120.26 g of the aqueous dispersion prepared in the step 2, and while this was kept stirred at a liquid temperature of 30°C, 2.7 g of TEOS was dropwise added thereto taking 420 minutes. After the dropwise addition, this was kept stirred for further 17 hours, and then cooled to form a second shell encapsulating the first shell, thereby giving an aqueous dispersion containing silica capsules (A-2) where the model fragrance material A was encapsulated with amorphous silica. The median diameter D₅₀ of the silica capsules (A-2) was 2.2 µm.

### Example 3

### (Step 1)

0.42 g of ΓaUARTAMIN 60W was diluted with 99.58 g of ion-exchanged water to prepare an aqueous phase component. An oily phase component prepared by mixing 40 g of the model fragrance material A and 10 g of TEOS was added to the aqueous phase component, and the mixture liquid was emulsified using the homomixer set at a rotation number of 8,500 rpm to give an emulsified liquid. At that time, the median diameter D₅₀ of the emulsified drops was 1.1 µm.

### (Step 2)

The emulsified liquid prepared in the step 1 was controlled to have a pH of 3.8 using 0.44 g of an aqueous solution of 0.1 N sodium hydroxide, then transferred to a separable flask equipped with a stirring blade and a cooling device, and while the liquid temperature was kept at 30°C, this was stirred at 160 rpm for 24 hours to give an aqueous dispersion containing silica capsules (1-3) each having a core of the model fragrance material A and a first shell of silica.

### (Step 3)

64.6 g was sampled from the total, 150.44 g of the aqueous dispersion prepared in the step 2, and while this was kept stirred at a liquid temperature of 30°C, 2.0 g of TEOS was dropwise added thereto taking 420 minutes. After the dropwise addition, this was kept stirred for further 17 hours, and then cooled to form a second shell encapsulating the first shell, thereby giving an aqueous dispersion containing silica capsules (A-3) where the model fragrance material A was encapsulated with amorphous silica.

### Example 4

### (Step 1)

0.42 g of ΓaUARTAMIN 60W was diluted with 99.58 g of ion-exchanged water to prepare an aqueous phase component. An oily phase component prepared by mixing 40 g of the model fragrance material A and 10 g of TEOS was added to the aqueous phase component, and the mixture liquid was emulsified using the homomixer set at a rotation number of 8,500 rpm to give an emulsified liquid. At that time, the median diameter D₅₀ of the emulsified drops was 1.1 µm. 120 g was sampled from the total, 150 g of the emulsified liquid, and diluted with 32 g of ion-exchanged water added thereto.

### (Step 2)

The emulsified liquid diluted in the step 1 was controlled to have a pH of 3.8 using 0.36 g of an aqueous solution of 0.1 N sodium hydroxide, then transferred to a separable flask equipped with a stirring blade and a cooling device, and while the liquid temperature was kept at 30°C, this was stirred at 160 rpm for 24 hours to give an aqueous dispersion containing silica capsules (1-4) each having a core of the model fragrance material A and a first shell of silica.

### (Step 3)

64.6 g was sampled from the total, 152.36 g of the aqueous dispersion prepared in the step 2, and while this was kept stirred at a liquid temperature of 30°C, 2.0 g of TEOS was dropwise added thereto taking 420 minutes. After the dropwise addition, this was kept stirred for further 17 hours, and then cooled to form a second shell encapsulating the first shell, thereby giving an aqueous dispersion containing silica capsules (A-4) where the model fragrance material A was encapsulated with amorphous silica. The median diameter D₅₀ of the silica capsules (A-4) was 2.2 µm.

### Example 5

### (Step 1)

0.50 g of ΓaUARTAMIN 60W was diluted with 99.51 g of ion-exchanged water to prepare an aqueous phase component. An oily phase component prepared by mixing 40 g of the model fragrance material B and 10 g of TEOS was added to the aqueous phase component, and the mixture liquid was emulsified using the homomixer set at a rotation number of 8,500 rpm to give an emulsified liquid. At that time, the median diameter D₅₀ of the emulsified drops was 1.1 µm. 120 g was sampled from the total, 150.01 g of the emulsified liquid, and diluted with 32 g of ion-exchanged water added thereto.

### (Step 2)

The emulsified liquid diluted in the step 1 was controlled to have a pH of 3.7 using 0.09 g of an aqueous solution of 0.5 N hydrochloric acid, then transferred to a separable flask equipped with a stirring blade and a cooling device, and while the liquid temperature was kept at 30°C, this was stirred at 200 rpm for 24 hours to give an aqueous dispersion containing silica capsules (1-5) each having a core of the model fragrance material B and a first shell of silica.

### (Step 3)

64.6 g was sampled from the total, 152.09 g of the aqueous dispersion prepared in the step 2, and while this was kept stirred at a liquid temperature of 30°C, 2.0 g of TEOS was dropwise added thereto taking 420 minutes. After the dropwise addition, this was kept stirred for further 17 hours, and then cooled to form a second shell encapsulating the first shell, thereby giving an aqueous dispersion containing silica capsules (B-5) where the model fragrance material B was encapsulated with amorphous silica. The median diameter D₅₀ of the silica capsules (B-5) was 2.2 µm.

### Comparative Example 1

### (Step 1)

7.14 g of ΓaUARTAMIN 60W was diluted with 392.84 g of ion-exchanged water to prepare an aqueous phase component. An oily phase component prepared by mixing 160 g of the model fragrance material A and 40 g of TEOS was added to the aqueous phase component, and the mixture liquid was emulsified using the homomixer set at a rotation number of 6,400 rpm to give an emulsified liquid. At that time, the median diameter D₅₀ of the emulsified drops was 2.0 µm.

### (Step 2)

The emulsified liquid prepared in the step 1 was controlled to have a pH of 3.8 using 1.73 g of an aqueous solution of 0.1 N sodium hydroxide, then transferred to a separable flask equipped with a stirring blade and a cooling device, and while the liquid temperature was kept at 30°C, this was stirred at 160 rpm for 24 hours to give an aqueous dispersion containing silica capsules (1-C1) each having a core of the model fragrance material A and a first shell of silica.

### (Step 3)

400.0 g was sampled from the total, 601.71 g of the aqueous dispersion prepared in the step 2, and while this was kept stirred at a liquid temperature of 30°C, 12.0 g of TEOS was dropwise added thereto taking 420 minutes. After the dropwise addition, this was kept stirred for further 17 hours, and then cooled to form a second shell encapsulating the first shell, thereby giving an aqueous dispersion containing silica capsules (A-C1) where the model fragrance material A was encapsulated with amorphous silica.

### Comparative Example 2

In the same manner as in Example 4, except that the step 3 was omitted in Example 4, an aqueous dispersion containing silica capsules (1-4) was prepared. The aqueous dispersion containing silica capsules (1-4) was used in the following evaluation. The median diameter D₅₀ of the silica capsules (1-4) was 2.2 µm.

### Comparative Example 3

In the same manner as in Example 5, except that the step 3 was omitted in Example 5, an aqueous dispersion containing silica capsules (1-5) was prepared. The aqueous dispersion containing silica capsules (1-5) was used in the following evaluation. The median diameter D₅₀ of the silica capsules (1-5) was 2.2 µm.

### Comparative Example 4

### (Step 1)

0.20 g of QUARTAMIN 60W and 0.60 g of a polyvinyl alcohol (tradename "Gohsenol GH20", by Nihon Gosei Kako Co., Ltd.) (hereinafter expressed as "PVA") were diluted with 119.20 g of ion-exchanged water to prepare an aqueous phase component. An oily phase component prepared by mixing 24 g of the model fragrance material B and 6 g of TEOS was added to the aqueous phase component, and the mixture liquid was emulsified using the homomixer set at a rotation number of 8,500 rpm to give an emulsified liquid. At that time, the median diameter D₅₀ of the emulsified drops was 0.7 µm.

### (Step 2)

The emulsified liquid prepared in the step 1 was controlled to have a pH of 3.7 using 0.17 g of an aqueous solution of 0.2 N hydrochloric acid, then transferred to a separable flask equipped with a stirring blade and a cooling device, and while the liquid temperature was kept at 30°C, this was stirred at 200 rpm for 24 hours to give an aqueous dispersion containing silica capsules (1-C4) each having a core of the model fragrance material B and a first shell of silica.

### (Step 3)

90.0 g was sampled from the total, 150.17 g of the aqueous dispersion prepared in the step 2, and while this was kept stirred at a liquid temperature of 30°C, 2.7 g of TEOS was dropwise added thereto taking 420 minutes. After the dropwise addition, this was kept stirred for further 17 hours, and then cooled to form a second shell encapsulating the first shell, thereby giving an aqueous dispersion containing silica capsules (B-C4) where the model fragrance material B was encapsulated with amorphous silica. The median diameter D₅₀ of the silica capsules (B-C4) was 2.1 µm.

### [Evaluation]

### [Evaluation of long-term retentiveness of organic compound encapsulated in microcapsules]

### (1) Preparation of softener for evaluation

Each aqueous dispersion containing silica capsules formed in Examples and Comparative Examples was added to a softener base material having the composition shown in the following Table 4 to prepare a softener for evaluation. The content of the encapsulated fragrance material was controlled to be 0.5% by mass.

**Table 4**

| Softener Base Material(*1) | Amount blended (part by mass) |
|---|---|
| Cationic softener base material(*2) | 12 |
| Polyoxyethylene(40) lauryl ether | 3.5 |
| Calcium chloride | 0.2 |
| Ethylene glycol | 1.6 |
| Proxel BDN(*3) | 0.01 |
| Methyl glycine diacetate trisodium | 0.01 |
| Ion-exchanged water | 82.68 |
| Total | 100 |

| | |
|---|---|
| Notes in Table 4 are as follows. *1: Added to make pH of softener base material 3.2. *2: Ester amine prepared by reacting plant fatty acid and triethanol amine in a ratio of 1.65/1 mol was quaternized with dimethyl sulfate in a known method. *3: by LONZA Japan, Ltd. | |

### (2) Evaluation method for long-term retentiveness of fragrance material component

The softener for evaluation prepared in the above (1) was put into a screw tube, sealed up therein and stored at 40°C. Immediately after the start of storage and after storage for a long period of time (14 days after the start of storage), the fragrance material retention rate of the fragrance material component was measured according to the following method. The results are shown in Table 5 to Table 6. A higher fragrance material retention rate after long-term storage indicates more excellent long-term retentiveness.

### [Measurement of fragrance material retention rate of fragrance material component]

Immediately after the start of storage or 14 days after the start of storage, the screw tube was taken out, and 100 mg of the softener for evaluation was scooped out with a syringe, diluted with 10 g of ion-exchanged water, and then made to pass through a membrane filter (by Millipore Corporation, tradename "Omnipore", Model Code "JAWP04700") to collect the silica capsules on the membrane filter.

Further, on the membrane filter, the silica capsules were washed with 10 mL of ion-exchanged water and then with 10 mL of hexane, and the silica capsules were immersed in 2 mL of acetonitrile containing an internal standard, tridecane in a concentration of 10 µg/ml, and irradiated with ultrasonic waves for 60 minutes using an ultrasonic irradiator (by Branson Corporation, Model "5510") under the condition of an output of 180 W and an oscillation frequency of 42 kHz to thereby elute the fragrance material out of the silica capsules. The resultant solution was again made to pass through a membrane filter (by Toyo Roshi Kaisha, Ltd., tradename "DISMIC", Model Code "13JP020AN"), and thereafter the fragrance material component contained in the solution was identified through gas chromatography to determine the amount α of the fragrance material component encapsulated in the silica capsules. The fragrance material retention rate was calculated according to the following formula.Fragrance material retention rate (%) = {(amount α of fragrance material component encapsulated in silica capsules immediately after start of storage or after long-term storage)/(amount β of fragrance material component contained in 100 mg of softener)} × 100

The amount β of the fragrance material component in the above formula was calculated from the formulation of the model fragrance material, the encapsulation ratio of each fragrance material component, and the blending amount of the silica capsules used in preparing the softener.

**Table 5**

| | Number of Silica Capsules | Step 1 | | | | | | Step 2 | Step 3 | Evaluation of Long-term Retentiveness in Softener | | | | | |
|---|---|---|---|---|---|---|---|---|---|---|---|---|---|---|---|
| | | Aqueous Phase Component | | | Oily Phase Component | | Emulsified Liquid | Initial pH of sol-gel reaction | Amount added of TEOS (mass%)* 2 | Fragrance Material retention rate (mass%) | | | | | |
| | | Kind of Emulsifi er | Content of emulsifier in aqueous phase component (mass%) | Content of cationic surfactant in emulsifier (mass%) | Kind of Organic Compound | Amount added of TEOS (mass%)*1 | Median diameter of emulsified drops D₅₀ (pm) | | | Immediately after start of storage (storage period at 40°C: 0 day) | | | After long-term storage (storage period at 40°C: 14 days) | | |
| | | | | | | | | | | Methyl dihydrojas monate | Tetrahydr olinalol | Hexyl salicylate | Methyl dihydroja smonate | Tetrahyd rolinalol | Hexyl salicylate |
| Example 1 | A-1 | CTAC | 0.20 | 100 | Model fragrance material A | 25 | 1.1 | 3.8 | 15 | 94 | 96 | 97 | 91 | 85 | 100 |
| Example 2 | A-2 | CTAC | 0.30 | 100 | Model fragrance material A | 25 | 1.1 | 3.8 | 15 | 100 | 100 | 100 | 85 | 77 | 100 |
| Example 3 | A-3 | CTAC | 0.13 | 100 | Model fragrance material A | 25 | 1.1 | 3.8 | 15 | 99 | 96 | 97 | 67 | 79 | 70 |
| Example 4 | A-4 | CTAC | 0.13 | 100 | Model fragrance material A | 25 | 1.1 | 3.8 | 15 | 99 | 97 | 100 | 69 | 64 | 86 |
| Compara tive Example 1 | A-C1 | CTAC | 0.54 | 100 | Model fragrance material A | 25 | 2.0 | 3.8 | 15 | 81 | 70 | 86 | 43 | 44 | 49 |
| Compara tive Example 2 | 1-4 | CTAC | 0.13 | 100 | Model fragrance material A | 25 | 1.1 | 3.8 | - | 86 | 80 | 89 | 28 | 18 | 51 |

| | | | | | | | | | | | | | | | |
|---|---|---|---|---|---|---|---|---|---|---|---|---|---|---|---|
| *1: Amount added of TEOS in the step 1 relative to the amount of the organic compound in the step 1 (mass%). *2: Amount added of TEOS in the step 3 relative to the amount of the organic compound in the step 1 (mass%). | | | | | | | | | | | | | | | |

**Table 6**

| | Number of Silica Capsules | Step 1 | | | | | | Step 2 | Step 3 | Evaluation of Long-term Retentiveness in Softener | | | | | |
|---|---|---|---|---|---|---|---|---|---|---|---|---|---|---|---|
| | | Aqueous Phase Component | | | Oily Phase Component | | Emulsified Liquid | Initial pH of sol-gel reaction | Amount added of TEOS (mass%)* 2 | Fragrance Material retention rate (mass%) | | | | | |
| | | Kind of Emulsi fier | Content of emulsifier in aqueous phase component (mass%) | Content of cationic surfactant in emulsifier (mass%) | Kind of Organic Compound | Amount added of TEOS (mass%)* 1 | Median diameter of emulsified drops D50 (pm) | | | Immediately after start of storage (storage period at 40°C: 0 day) | | | After long-term storage (storage period at 40°C: 14 days) | | |
| | | | | | | | | | | Total of 1,8-cineol and limonene* 4 | Total of linalool and tetrahydr olinalool | Linalyl acetate | Total of 1,8-cineol and limonene *4 | Total of linalool and tetrahydr olinalool | Linalyl acetate |
| Example 5 | B-5 | CTAC | 0.15 | 100 | Model fragrance material B | 25 | 1.1 | 3.7 | 15 | 93 | 94 | 100 | 77 | 99 | 76 |
| Compara tive Example 3 | 1-5 | CTAC | 0.15 | 100 | Model fragrance material B | 25 | 1.1 | 3.7 | - | 81 | 90 | 93 | 13 | 12 | 40 |
| Compara tive Example 4 | B-C4 | CTAC /PVA | 0.55 *3 | 9 | Model fragrance material B | 25 | 0.7 | 3.7 | 15 | 3 | 0 | 0 | - | - | - |

| | | | | | | | | | | | | | | | |
|---|---|---|---|---|---|---|---|---|---|---|---|---|---|---|---|
| *1: Amount added of TEOS in the step 1 relative to the amount of the organic compound in the step 1 (mass%). *2: Amount added of TEOS in the step 3 relative to the amount of the organic compound in the step 1 (mass%). *3: Total of the content of CTAC in the aqueous phase component, 0.05% by mass, and the content of PVAin the aqueous phase component, 0.50% by mass. *4: Derived from eucalyptus oil. | | | | | | | | | | | | | | | |

From Table 5 and Table 6, it is known that, in the silica capsules formed in Examples 1 to 5, more than half of the blended fragrance material components were retained in the silica capsules even after long-term storage, that is, the silica capsules are excellent in long-term retentiveness, as compared with those in Comparative Examples 1 to 4.

### [Evaluation of dispersion stability of microcapsules against alkali]

0.2 g of an aqueous solution of 0.1 N sodium hydroxide was added to 2 g of the aqueous dispersion containing silica capsules formed in Examples 1 to 3 and Comparative Example 1, and then held at room temperature to check for the presence or absence of flowability of the dispersion. In Examples 1 to 3, the dispersion did not gel and kept flowability. On the other hand, in Comparative Example 1, the dispersion geld and lost flowability within 30 minutes after addition of the aqueous solution of sodium hydroxide.

From this, it is known that the silica capsules produced according to the production method of the present invention have high dispersion stability against alkali.

### Industrial Applicability

The silica capsules produced according to the production method of the present invention are excellent in long-term retentiveness of an organic compound that is an active component of fragrance materials, and have high dispersion stability against alkali, and therefore can be stably blended in any production for acidic preparations, neutral preparations and alkaline preparations.

## Claims

1. A method for producing microcapsules each having a shell that comprises silica as a constituent component, and a core that comprises at least one organic compound inside the shell, which comprises:
Step 1: a step of emulsifying an aqueous phase component comprising an emulsifier and an oily phase component comprising at least one organic compound and a tetraalkoxysilane to give an emulsified liquid,
Step 2: a step of subjecting the emulsified liquid prepared in the step 1 to a sol-gel reaction under an acidic condition to form microcapsules (1) each having a core and a first shell comprising silica as a constituent component, thereby giving an aqueous dispersion comprising the microcapsules (1), and
Step 3: a step of further adding a tetraalkoxysilane to the aqueous dispersion comprising the microcapsules (1) formed in the step 2, and subjecting the obtained mixture to sol-gel reaction to form microcapsules each having a second shell that encapsulates the first shell, wherein:
the emulsifier used in the step 1 comprises a cationic surfactant, and
the content of the emulsifier in the aqueous phase component in the step 1 is 0.30% by mass or less.

2. The method for producing microcapsules according to claim 1, wherein the content of the cationic surfactant in the emulsifier in the step 1 is 90% by mass or more.

3. The method for producing microcapsules according to claim 1 or 2, wherein the cationic surfactant is an alkyltrimethylammonium salt.

4. The method for producing microcapsules according to any of claims 1 to 3, wherein the cationic surfactant is cetyltrimethylammonium chloride.

5. The method for producing microcapsules according to any of claims 1 to 4, wherein the content of the cationic surfactant in the aqueous phase component in the step 1 is 0.20% by mass or less.

6. The method for producing microcapsules according to any of claims 1 to 5, wherein the median diameter D₅₀ of the emulsified drops in the emulsified liquid prepared in the step 1 is 0.1 µm or more and 10 µm or less.

7. The method for producing microcapsules according to any of claims 1 to 6, wherein the amount to be added of tetraalkoxysilane in the step 1 is 10% by mass or more and 100% by mass or less relative to the amount of the organic compound in the step 1.

8. The method for producing microcapsules according to any of claims 1 to 7, wherein the amount to be added of tetraalkoxysilane in the step 3 is 10% by mass or more and 100% by mass or less relative to the amount of the organic compound in the step 1.

9. The method for producing microcapsules according to any of claims 1 to 8, wherein the initial pH in the sol-gel reaction in the step 2 is 3.0 or more and 4.5 or less.

10. The method for producing microcapsules according to any of claims 1 to 9, wherein the organic compound comprised in the core is at least one material selected from the group consisting of a fragrance material, a fragrance precursor, an oil, an antioxidant, an antibacterial agent, a fertilizer, a surface modifier, and a solvent.

## Patentansprüche

1. Verfahren zur Herstellung von Mikrokapseln, die jeweils eine Hülle, umfassend Siliciumoxid als Bestandteil, und einen Kern, umfassend mindestens eine organische Verbindung, innerhalb der Hülle aufweisen, welches umfasst:
Schritt 1: einen Schritt des Emulgierens einer wässrigen Phasenkomponente, umfassend einen Emulgator, und einer öligen Phasenkomponente, umfassend mindestens eine organische Verbindung und ein Tetraalkoxysilan, um eine emulgierte Flüssigkeit zu erhalten,
Schritt 2: einen Schritt des Unterziehens der in Schritt 1 hergestellten emulgierten Flüssigkeit einer Sol-Gel-Reaktion unter sauren Bedingungen, um Mikrokapseln (1) zu bilden, die jeweils einen Kern und eine erste Hülle, umfassend Siliciumoxid als Bestandteil, aufweisen, wodurch eine wässrige Dispersion, umfassend die Mikrokapseln (1), erhalten wird, und
Schritt 3: einen Schritt des weiteren Hinzufügens eines Tetraalkoxysilans zu der in Schritt 2 gebildeten wässrigen Dispersion, umfassend die Mikrokapseln (1), und des Unterziehen der erhaltenen Mischung einer Sol-Gel-Reaktion, um Mikrokapseln zu bilden, die jeweils eine zweite Hülle aufweisen, die die erste Hülle einkapselt, wobei:
der in Schritt 1 verwendete Emulgator ein kationisches Tensid umfasst, und
der Gehalt des Emulgators in der wässrigen Phasenkomponente in Schritt 1 0,30 Massen-% oder weniger beträgt.

2. Verfahren zur Herstellung von Mikrokapseln gemäß Anspruch 1, wobei der Gehalt des kationischen Tensids in dem Emulgator in Schritt 1 90 Massen-% oder mehr beträgt.

3. Verfahren zur Herstellung von Mikrokapseln gemäß Anspruch 1 oder 2, wobei das kationische Tensid ein Alkyltrimethylammoniumsalz ist.

4. Verfahren zur Herstellung von Mikrokapseln gemäß einem der Ansprüche 1 bis 3, wobei das kationischen Tensid Cetyltrimethylammoniumchlorid ist.

5. Verfahren zur Herstellung von Mikrokapseln gemäß einem der Ansprüche 1 bis 4, wobei der Gehalt des kationischen Tensids in der wässrigen Phasenkomponente in Schritt 1 0,20 Massen-% oder weniger beträgt.

6. Verfahren zur Herstellung von Mikrokapseln gemäß einem der Ansprüche 1 bis 5, wobei der mediane Durchmesser D₅₀ der emulgierten Tropfen in der in Schritt 1 hergestellten emulgierten Flüssigkeit 0,1 um oder mehr und 10 µm oder weniger beträgt.

7. Verfahren zur Herstellung von Mikrokapseln gemäß einem der Ansprüche 1 bis 6, wobei die in Schritt 1 zuzusetzende Menge an Tetraalkoxysilan 10 Massen-% oder mehr und 100 Massen-% oder weniger, bezogen auf die Menge der organischen Verbindung in Schritt 1, beträgt.

8. Verfahren zur Herstellung von Mikrokapseln gemäß einem der Ansprüche 1 bis 7, wobei die zuzusetzende Menge an Tetraalkoxysilan in Schritt 3 10 Massen-% oder mehr und 100 Massen-% oder weniger, bezogen auf die Menge der organischen Verbindung in Schritt 1, beträgt.

9. Verfahren zur Herstellung von Mikrokapseln gemäß einem der Ansprüche 1 bis 8, wobei der anfängliche pH-Wert bei der Sol-Gel-Reaktion in Schritt 2 3,0 oder mehr und 4,5 oder weniger beträgt.

10. Verfahren zur Herstellung von Mikrokapseln gemäß einem der Ansprüche 1 bis 9, wobei die in dem Kern enthaltene organische Verbindung mindestens ein Material ist, das aus der Gruppe ausgewählt ist, die aus einem Duftstoffmaterial, einem Duftstoffvorläufer, einem Öl, einem Antioxidans, einem antibakteriellen Mittel, einem Düngemittel, einem Oberflächenmodifikator und einem Lösungsmittel besteht.

## Revendications

1. Procédé de production de microcapsules présentant chacune une enveloppe qui comprend de la silice en tant que composant constitutif, et un coeur qui comprend au moins un composé organique à l'intérieur de l'enveloppe, qui comprend :
Étape 1 : une étape d'émulsification d'un composant de phase aqueuse comprenant un émulsifiant et un composant de phase huileuse comprenant au moins un composé organique et un tétraalcoxysilane pour donner un liquide émulsifié,
Étape 2 : une étape de soumission du liquide émulsifié préparé dans l'étape 1 à une réaction sol-gel dans des conditions acides pour former des microcapsules (1) présentant chacune un coeur et une première enveloppe comprenant de la silice en tant que composant constitutif, donnant ainsi une dispersion aqueuse comprenant les microcapsules (1), et
Étape 3 : une étape d'ajout supplémentaire d'un tétraalcoxysilane à la dispersion aqueuse comprenant les microcapsules (1) formées dans l'étape 2, et de soumission du mélange obtenu à une réaction sol-gel pour former des microcapsules présentant chacune une deuxième enveloppe qui encapsule la première enveloppe, dans lequel :
l'émulsifiant utilisé dans l'étape 1 comprend un tensioactif cationique, et
la teneur de l'émulsifiant dans le composant de phase aqueuse dans l'étape 1 est de 0,30 % en masse ou moins.

2. Procédé de production de microcapsules selon la revendication 1, dans lequel la teneur du tensioactif cationique dans l'émulsifiant dans l'étape 1 est de 90 % en masse ou plus.

3. Procédé de production de microcapsules selon la revendication 1 ou la revendication 2, dans lequel le tensioactif cationique est un sel d'alkyltriméthylammonium.

4. Procédé de production de microcapsules selon l'une quelconque des revendications 1 à 3, dans lequel le tensioactif cationique est du chlorure de cétyltriméthylammonium.

5. Procédé de production de microcapsules selon l'une quelconque des revendications 1 à 4, dans lequel la teneur du tensioactif cationique dans le composant de phase aqueuse dans l'étape 1 est de 0,20 % en masse ou moins.

6. Procédé de production de microcapsules selon l'une quelconque des revendications 1 à 5, dans lequel le diamètre médian D₅₀ des gouttes émulsifiées dans le liquide émulsifié préparé dans l'étape 1 est de 0,1 µm ou plus et de 10 µm ou moins.

7. Procédé de production de microcapsules selon l'une quelconque des revendications 1 à 6, dans lequel la quantité à ajouter de tétraalcoxysilane dans l'étape 1 est de 10 % en masse ou plus et de 100 % en masse ou moins par rapport à la quantité du composé organique dans l'étape 1.

8. Procédé de production de microcapsules selon l'une quelconque des revendications 1 à 7, dans lequel la quantité à ajouter de tétraalcoxysilane dans l'étape 3 est de 10 % en masse ou plus et de 100 % en masse ou moins par rapport à la quantité du composé organique dans l'étape 1.

9. Procédé de production de microcapsules selon l'une quelconque des revendications 1 à 8, dans lequel le pH initial dans la réaction sol-gel dans l'étape 2 est de 3,0 ou plus et de 4,5 ou moins.

10. Procédé de production de microcapsules selon l'une quelconque des revendications 1 à 9, dans lequel le composé organique compris dans le coeur est au moins un matériau sélectionné dans le groupe consistant en un matériau de parfum, un précurseur de parfum, une huile, un antioxydant, un agent antibactérien, un engrais, un modificateur de surface et un solvant.
